(19) Europäisches Patentamt
European Patent Office
Office européen des brevets

(11) Veröffentlichungsnummer: **0 007 588**
**B1**

(12) # EUROPÄISCHE PATENTSCHRIFT

(45) Veröffentlichungstag der Patentschrift: **13.05.81**

(21) Anmeldenummer: **79102556.2**

(22) Anmeldetag: **20.07.79**

(51) Int. Cl.³: **C 07 D 265/06,** A 01 N 43/86, C 07 D 207/327, C 07 D 231/12, C 07 D 233/61, C 07 D 249/04, C 07 D 249/08, C 07 D 257/04

(54) **Tetrahydro-1,3-oxazine, herbizide Mittel, die Acetanilide als herbizide Wirkstoffe und diese Tetrahydro-1,3-oxazine als antagonistische Mittel enthalten, sowie ihre Verwendung zur Bekämpfung unerwünschten Pflanzenwuchses.**

(30) Priorität: **27.07.78 DE 2832890**

(43) Veröffentlichungstag der Anmeldung: **06.02.80 Patentblatt 80/3**

(45) Bekanntmachung des Hinweises auf die Patenterteilung: **13.05.81 Patentblatt 81/19**

(84) Benannte Vertragsstaaten: **AT BE CH DE FR GB IT LU NL SE**

(56) Entgegenhaltungen:
**DE-A-2 218 097**
**DE-A-2 221 408**
**DE-A-2 402 983**
**DE-A-2 648 008**
**US-A-3 719 466**

(73) Patentinhaber: **BASF Aktiengesellschaft, Carl-Bosch-Strasse 38, D-6700 Ludwigshafen (DE)**

(72) Erfinder: **Eicken, Karl, Dr.-Chem., Waldstrasse 63, D-6706 Wachenheim (DE)**
Erfinder: **Rohr, Wolfgang, Dr.-Chem., Gontardstrasse 4, D-6800 Mannheim 1 (DE)**
Erfinder: **Pander, Hans Joachim, Hochdorfer Strasse 19, D-6701 Roedersheim-Gronau 2 (DE)**
Erfinder: **Wuerzer, Bruno, Dr., Wilhelm-Busch-Strasse 55, D-6703 Limburgerhof (DE)**

**EP 0 007 588 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

ACTORUM AG.

# Tetrahydro-1,3-oxazine, herbizide Mittel, die Acetanilide als herbizide Wirkstoffe und diese Tetrahydro-1,3-oxazine als antagonistische Mittel enthalten, sowie ihre Verwendung zur Bekämpfung unerwünschten Pflanzenwuchses

Die vorliegende Erfindung betrifft neue Tetrahydro-1,3-oxazine, sowie herbizide Mittel, die substituierte Acetanilide als herbizide Wirkstoffe und diese Tetrahydro-1,3-oxazine als antagonistische Mittel enthalten, sowie Verfahren zur selektiven Bekämpfung von unerwünschtem Pflanzenwuchs mit diesen herbiziden Mitteln.

Substituierte Acetanilide der Formel I

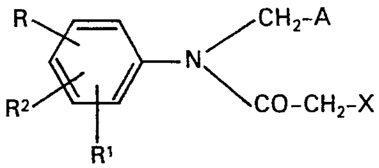

I

in der

R Wasserstoff, einen unverzweigten oder verzweigten Alkyl- oder Alkoxyrest mit bis zu 5 Kohlenstoffatomen,

$R^1$ Wasserstoff, Halogen, einen unverzweigten oder verzweigten Alkyl- oder Alkoxyrest mit bis zu 5 Kohlenstoffatomen,

$R^2$ Wasserstoff, Halogen, einen unverzweigten oder verzweigten Alkyl- oder Alkoxyrest mit bis zu 5 Kohlenstoffatomen,

R zusammen mit $R^2$ eine orthoständig verknüpfte, gegebenenfalls durch unverzweigte oder verzweigte Alkylgruppen mit bis zu 4 Kohlenstoffatomen substituierte Alkylenketten mit bis zu 6 Kohlenstoffatomen,

X Chlor oder Brom und

A ein über ein Ringstickstoffatom gebundenes Azol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy, Carbalkoxy mit bis zu 4 Kohlenstoffatomen in der Alkoxygruppe oder Alkanoylreste mit bis zu 4 Kohlenstoffatomen substituiert sein kann, bedeutet, wobei A auch für Salze der 2 oder 3 Stickstoffatome enthaltenden Azole stehen kann, sind herbizid ausgezeichnet wirksam, führen aber bei Anwendung in Kulturen, wie Mais, oder in Kulturen aus der Familie der Gramineen zu Schädigungen der Nutzpflanzen.

Aufgabe der Erfindung war es deshalb, antagonistische Mittel zu finden, die diese Unverträglichkeit der herbiziden Acetanilide gegenüber bestimmten Kulturpflanzen kompensieren.

Herbizide Mittel, die neben Chloracetaniliden als herbiziden Wirkstoffen antagonistisch wirkende Verbindungen enthalten, sind aus der US-A-3719466, der DE-A-2218097 und der DE-A-2402983 bekannt.

Die US-A-3719466 lehrt, dass Schädigungen an Sorghum und Weizen, die durch das Herbizid 2-Chlor-2′,6′-diäthyl-N-(methoxymethyl)-acetanilid hervorgerufen werden, durch Behandlung des Saatgutes mit einem antagonistischen Mittel, beispielsweise N,N-Diallyl-acetamid, vermieden werden können.

In der DE-A-2218097 werden Kombinationen aus dem gleichen Wirkstoff sowie anderen herbiziden Acetaniliden und antagonistisch wirkenden Amiden, beispielsweise N,N-Diallyl-dichloracetamid, erwähnt. Diese Amide werden allerdings bevorzugt als Antidots für herbizide Thiolcarbamate verwendet.

Gegenstand der DE-A-2402983 sind herbizide Mittel, die aus der DE-A-2218097 bereits bekannte oder diesen strukturell ähnliche Dichloracetamide zusammen mit Chloracetaniliden anderer Konstitution, insbesondere N-(2′-Methoxyäthyl)-2,6-dimethyl-chloracetanilid, enthalten. Diese Mittel eignen sich lediglich zur selektiven Unkrautbekämpfung in Mais. Unter anderem kommen als antagonistische Verbindungen Dichloracetamide, bei denen die beiden Substituenten am Stickstoffatom zusammen mit diesem einen 6-gliedrigen Heterocyclus, der noch ein weiteres Heteroatom enthalten und durch niedere Alkylreste einfach oder mehrfach substituiert sein kann, bilden, in Betracht. Als Vertreter dieser Gruppe ist jedoch lediglich N-Dichloracetyl-morpholin genannt.

Es wurde gefunden, dass neue Tetrahydro-1,3-oxazine der Formel II

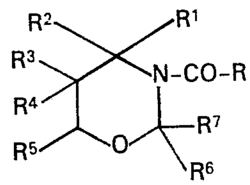

II

in der

R einen unverzweigten oder verzweigten Halogenalkylrest mit bis zu 3 Kohlenstoffatomen,

$R^1$, $R^2$, $R^3$, $R^4$ und $R^5$ gleich oder verschieden sind und Wasserstoff oder einen unverzweigten oder verzweigten Alkylrest mit bis zu 3 Kohlenstoffatomen,

$R^6$ Wasserstoff oder einen unverzweigten oder verzweigten Alkylrest mit bis zu 8 Kohlenstoffatomen,

$R^7$ Wasserstoff, einen unverzweigten oder verzweigten Alkylrest mit bis zu 8 Kohlenstoffatomen, einen Alkoxyalkylrest mit bis zu 6 Kohlenstoffatomen oder einen Dialkoxyalkylrest mit bis zu 8 Kohlenstoffatomen bedeuten und

$R^6$ und $R^7$ zusammen eine Methylenkette mit 4 oder 5 Kohlenstoffatomen bilden können,

sich ausgezeichnet zur Steigerung der Kulturpflanzenverträglichkeit von herbiziden substituierten Acetaniliden der Formel I eignen. Herbizide Mittel, die mindestens ein substituiertes Acetanilid der Formel I und mindestens ein Tetrahydro-1,3-oxazin der Formel II enthalten, können sowohl in Mais als auch in Getreidekulturen eingesetzt werden. Dabei bleibt die gute herbizide Wirkung der Acetanilide erhalten, während Schädigungen an den Nutzpflanzen unterbunden werden.

Acetanilide, deren Kulturpflanzenverträglichkeit durch die neuen Tetrahydro-1,3-oxazine gesteigert werden kann, sind solche der Formel I, bei denen

R für Wasserstoff, Alkyl bis zu 5 Kohlenstoffatomen wie Methyl, Äthyl, n-Propyl, i-Propyl, n-Butyl, sec.-Butyl, i-Butyl, tert.-Butyl, normale und verzweigte Pentylreste, Alkoxy mit bis zu 5 Kohlenstoffatomen, wie Methoxy, Äthoxy, Propoxy, Butoxy, Pentyloxy;

$R^1$ und $R^2$ für Wasserstoff, Halogen, wie Fluor, Chlor, Brom oder Jod, Alkyl bis zu 5 Kohlenstoffatomen, wie Methyl, Äthyl, n-Propyl, i-Propyl, n-Butyl, sec.-Butyl, i-Butyl, tert.-Butyl, normale und verzweigte Pentylreste, Alkoxy mit bis zu 5 Kohlenstoffatomen, wie Methoxy, Äthoxy, Propoxy, Butoxy, Pentyloxy;

R zusammen mit $R^2$ für eine orthoständig verknüpfte, gegebenenfalls durch Alkyl mit bis zu 4 Kohlenstoffatomen substituierte Alkylenkette mit bis zu 6 Kohlenstoffatomen, wie Äthylen, Trimethylen, Tetramethylen, 1-Methyl-trimethylen, 1,1-Dimethyl-trimethylen, 1,1-Dimethyl-tetramethylen;

X für Chlor oder Brom, vorzugsweise Chlor, und

A für ein über ein Ring-Stickstoffatom gebundenes Azol, wie Pyrrol, Pyrazol, Imidazol, 1,2,4-Triazol, 1,2,3-Triazol, Tetrazol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy, Carbalkoxyreste mit bis zu 4 Kohlenstoffatomen in der Alkoxygruppe oder Alkanoyl mit bis zu 4 Kohlenstoffatomen unabhängig voneinander substituiert sein kann, wie 2,6-Dimethylpyrrol,
Tetramethylpyrrol, 3(5)-Methylpyrazol,
4-Methylpyrazol, 3(5)-Äthylpyrazol,
4-Äthylpyrazol, 3(5)-Isopropylpyrazol,
4-Isopropylpyrazol, 3,5-Dimethylpyrazol,
3,5-Dimethyl-4-acetylpyrazol,
3,5-Dimethyl-4-propionylpyrazol,
3,4,5-Trimethylpyrazol, 3(5)-Phenylpyrazol,
4-Phenylpyrazol, 3,5-Diphenylpyrazol,
3(5)-Phenyl-5(3)-methylpyrazol,
3(5)-Chlorpyrazol, 4-Chlorpyrazol,
4-Brompyrazol, 4-Jodpyrazol,
3,4,5-Tribrompyrazol,
3,5-Dimethyl-4-chlorpyrazol,
3,5-Dimethyl-4-brompyrazol,
4-Chlor-3(5)-methylpyrazol,
4-Brom-3(5)-methylpyrazol,
4-Methyl-3,5-dichlorpyrazol,
3(5)-Methyl-4,5(3)-dichlorpyrazol,
3(5)-Chlor-5(3)-methylpyrazol,
4-Methoxypyrazol,
3(5)-Methyl-5(3)-methoxypyrazol,
3(5)-Äthoxy-4,5(3)-dimethylpyrazol,
3(5)-Methyl-5(3)-trifluormethyl-pyrazol,
3,5-Bistrifluormethylpyrazol,
3(5)-Methyl-5(3)-carbäthoxypyrazol,
3,5-Biscarbäthoxypyrazol,
3,4,5-Triscarbäthoxypyrazol,
3(5)-Methyl-5(3)-methylthio-4-carbäthoxypyrazol, 4-Methyl-3,5-biscarbäthoxypyrazol,
4-Cyanopyrazol, 4-Methoxy-3,5-dichlorpyrazol,
4,5-Dichlor-imidazol, 2-Äthyl-4,5-dichlorimidazol,
2-Methyl-4,5-dichlorimidazol,
3(5)-Methyl-1,2,4-triazol,
3,5-Dimethyl-1,2,4-triazol,
3(5)-Chlor-1,2,4-triazol, 3(5)-Brom-1,2,4-triazol,
3(5)-Chlor-5(3)-methyl-1,2,4-triazol,
3,5-Dichlor-1,2,4-triazol, 3,5-Dibrom-1,2,4-triazol,
3(5)-Chlor-5(3)-cyano-1,2,4-triazol,
3(5)-Chlor-5(3)-phenyl-1,2,4-triazol,
3(5)-Chlor-5(3)-carbomethoxy-1,2,4-triazol,
3(5)-Methylthio-1,2,4-triazol,
4(5)-Methyl-1,2,3-triazol,
4,5-Dimethyl-1,2,3-triazol,
4(5)-Phenyl-1,2,3-triazol, 4(5)-Chlor-1,2,3-triazol,
1,2,3-Triazol-4(5)-yl-carbonsäureäthylester,
1,2,3-Triazol-4,5-yl-dicarbonsäuredimethylester,
5-Methyltetrazol, 5-Chlortetrazol,
Tetrazolyl-5-carbonsäureäthylester, steht.

Darüber hinaus kann der Rest A, wenn das gegebenenfalls substituierte Azol 2 oder 3 Stickstoffatome enthält, auch salzartig an eine der üblichen starken anorganischen oder organischen Säuren, wie Chlorwasserstoffsäure, Bromwasserstoffsäure, Salpetersäure, Schwefelsäure, Tetrafluorborsäure, Fluorsulfonsäure, Ameisensäure, eine halogenierte Carbonsäure, z.B. Trichloressigsäure, eine Alkansulfonsäure, z.B. Methansulfonsäure, eine halogenierte Alkansulfonsäure, z.B. Trifluormethansulfonsäure, Perfluorhexansulfonsäure, eine Arylsulfonsäure, z.B. Dodecylbenzolsulfonsäure, gebunden sein.

Bevorzugt sind Acetanilide, die in 2- und 6-Stellung am Phenylring Methyl oder Äthyl und in 3-Stellung Wasserstoff, Methyl oder Äthyl tragen, wobei als Azole Pyrazol, Imidazol, Triazol oder Tetrazol, die jeweils durch niedere Alkyl-, Alkoxy-, Alkylthioreste, Carbalkoxy, Cyan oder Halogen substituiert sein können, in Betracht kommen.

Insbesondere enthalten die erfindunsgemässen herbiziden Mittel folgende Acetanilide:
2-Chlor-2′,6′-dimethyl-N-(pyrazol-1-yl-methyl)-acetanilid,
2-Chlor-2′-methyl-6′-äthyl-N-(pyrazol-1-yl-methyl)-acetanilid,
2-Chlor-2′,6′-dimethyl-N-(4-methylpyrazol-1-yl-methyl)-acetanilid,
2-Chlor-2-methyl-6-äthyl-N-(4-methoxypyrazol-1-yl-methyl)-acetanilid,
2-Chlor-2′-methyl-6′-äthyl-N-(3(5)-methylpyrazol-1-yl)-acetanilid,
2-Chlor-2′,6′-dimethyl-N-(3,5-dimethylpyrazol-1-yl-methyl)-acetanilid,
2-Chlor-2′,6′-dimethyl-N-(1,2,4-triazol-1-yl-methyl)-acetanilid,
2-Chlor-2′,6′-dimethyl-N-(4-chlorpyrazol-1-yl-methyl)-acetanilid,
2-Chlor-2′,3′,6′-trimethyl-N-(pyrazol-1-yl-methyl)-acetanilid,
2-Chlor-2′-methyl-6′-äthyl-N-(3,5-dimethyl-pyrazol-1-yl-methyl)-acetanilid,
2-Chlor-2′,6′-diäthyl-N-(3,5-dimethyl-pyrazol-1-yl-methyl)-acetanilid,
2-Chlor-2′,3′,6′-trimethyl-N-(3,5-dimethyl-pyrazol-1-yl-methyl)-acetanilid,
2-Chlor-2′,6′-diäthyl-N-(4-methylpyrazol-1-yl-methyl)-acetanilid,

2-Chlor-2′-methyl-6′-äthyl-N-(4-methylpyrazol-1-yl-methyl)-acetanilid,
2-Chlor-2′,3′,6′-trimethyl-N-(4-methylpyrazol-1-yl-methyl)-acetanilid,
2-Chlor-2′,6′-dimethyl-N-(3-(5)-methylpyrazol-1-yl-methyl)-acetanilid,
2-Chlor-2′,6′-diäthyl-N-(3-(5)-methylpyrazol-1-yl-methyl)-acetanilid,
2-Chlor-2′,6′-dimethyl-N-(4-methoxypyrazol-1-yl-methyl)-acetanilid,
2-Chlor-2′,6′-diäthyl-N-(pyrazol-1-yl-methyl)-acetanilid,
2-Chlor-2′,6′-dimethyl-N-(4,5-dichlorimidazol-1-yl-methyl)-acetanilid,
2-Chlor-2′-methyl-6′-äthyl-N-(2-äthyl-4,5-dichlorimidazol-1-yl-methyl)-acetanilid,
2-Chlor-2′,6′-diäthyl-N-(4,5-dichlorimidazol-1-yl-methyl)-acetanilid,
2-Chlor-2′-methyl-6′-äthyl-N-(1,2,4-triazol-1-yl-methyl)-acetanilid,
2-Chlor-2′,6′-diäthyl-N-(1,2,4-triazol-1-yl-methyl)-acetanilid und
2-Chlor-2′,3′,6′-trimethyl-N-(1,2,4-triazol-1-yl-methyl)-acetanilid.

Die Acetanilide der Formel I sind Gegenstand der DE-A-2648008 und der deutschen Patentanmeldung P 2744396. Sie können durch Umsetzung von 2-Halogen-N-halogenmethylacetaniliden der Formel V mit einem 1H-Azol der Formel H-A nach folgender Reaktionsgleichung erhalten werden:

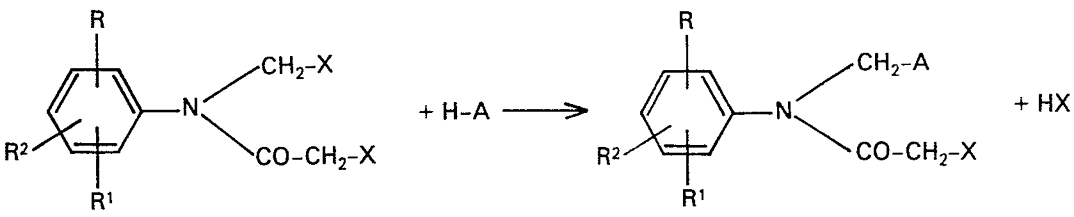


V I

Dabei haben die Substituenten R, R[1], R[2] und X die oben genannte Bedeutung und A steht für ein über ein Ring-Stickstoffatom gebundenes Azol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy, Carbalkoxyreste mit bis zu 4 Kohlenstoffatomen in der Alkoxygruppe oder Alkanoylreste mit bis zu 4 Kohlenstoffatomen substituiert sein kann.

Als antagonistische Mittel kommen Tetrahydro-1,3-oxazine der Formel II in Betracht, bei denen die Substituenten R[1] bis R[5] gleich oder verschieden sind und Wasserstoff oder Alkyl mit bis zu 3 Kohlenstoffatomen, insbesondere Wasserstoff oder Methyl, bedeuten. R[6] und R[7] bedeuten Wasserstoff oder Alkylreste mit bis zu 8 Kohlenstoffatomen, wie Methyl-, Äthyl, N-Propyl, i-Propyl, Butyl-, Hexyl-, Heptyl- oder Octylreste.

R[7] kann auch für Alkoxyalkylreste mit bis zu 6 Kohlenstoffatomen oder für Dialkoxyalkylreste mit bis zu 8 Kohlenstoffatomen stehen, beispielsweise für Methoxymethyl, Dimethoxymethyl. R[6] und R[7] können zusammen eine Methylenkette mit 4 oder 5 Kohlenstoffatomen bilden. R bedeutet Halogenalkyl mit bis zu 3 Kohlenstoffatomen, vorzugsweise Chloralkyl, insbesondere Chlormethyl oder Dichlormethyl.

Bevorzugte Tetrahydro-1,3-oxazine sind
N-Dichloracetyl-4,4-dimethyl-tetrahydro-1,3-oxazin und
N-Dichloracetyl-4,4,6-trimethyl-tetrahydro-1,3-oxazin.

Die neuen Tetrahydro-1,3-oxazine der Formel II werden durch Umsetzung einer Verbindung der Formel III

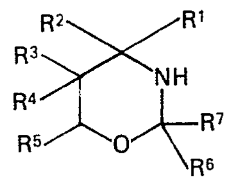

III,

in der R[1], R[2], R[3], R[4], R[5], R[6] und R[7] die obengenannten Bedeutungen haben, mit einem Säurechlorid der Formel IV

R-CO-Cl IV,

in der R die obengenannte Bedeutung hat, in Gegenwart eines Chlorwasserstoff-bindenden Mittels in einem inerten Lösungs- oder Verdünnungsmittel erhalten.

Chlorwasserstoff-bindende Mittel können anorganische Basen, wie Alkalicarbonate, Alkalihydrogencarbonate, Alkalihydroxide, oder organische Basen, beispielsweise tertiäre Amine, wie Trialkylamine, insbesondere Triäthylamin, sein.

Als inerte Lösungs- oder Verdünnungsmittel kommen Kohlenwasserstoffe, wie Toluol, Xylole, Ligroin, Cyclohexan, halogenierte Kohlenwasserstoffe, wie Dichlormethan, Chloroform, Tetrachlorkohlenstoff und Äther, wie Diäthyläther, Diisopropyläther, Tetrahydrofuran, Dioxan, Anisol, in Betracht.

Das folgende Beispiel erläutert die Herstellung der neuen Tetrahydro-1,3-oxazine. Gewichtsteile verhalten sich zu Volumenteilen wie Kilogramm zu Liter.

Beispiel 1

Zu 23,0 Gewichtsteilen 4,4-Dimethyl-tetrahydro-1,3-oxazin und 20,7 Gewichtsteilen Triäthylamin in 100 Volumenteilen Toluol werden bei -10 °C 23,2 Gewichtsteile Dichloracetylchlorid in 100 Volumenteilen Toluol unter Rühren zugetropft. Nach 2-stündigem Nachrühren bei Raumtemperatur werden 150 Volumenteile Methylenchlorid und so viel Wasser zugesetzt, dass zwei klare Phasen entstehen. Die organische Phase wird abgetrennt und zweimal mit je 50 Volumenteilen Wasser gewaschen. Nach dem Trocknen und Verdampfen der Lösungsmittel unter vermindertem Druck isoliert man 41 Gewichtsteile N-Dichloracetyl-4,4-dimethyl-tetrahydro-1,3-oxazin vom Fp. 105–106 °C, das nach Umkristallisieren aus Methanol bei 106–107 °C schmilzt.

$C_8H_{13}N_2O_2Cl_2$ MG 226

| | | | | |
|---|---|---|---|---|
| ber. | C 42,5 | H 5,8 | N 6,19 |
| gef.: | C 42,6 | H 5,8 | N 6,2 |

In analoger Weise können folgende Verbindungen hergestellt werden

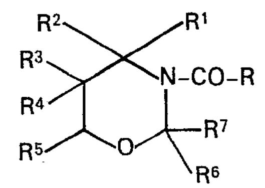

| Nr. | R | $R^1$ | $R^2$ | $R^3$ | $R^4$ | $R^5$ | $R^6$ | $R^7$ | Fp/Kp/$n_D^{25}$ |
|---|---|---|---|---|---|---|---|---|---|
| 1 | $CH_2Cl$ | $CH_3$ | $CH_3$ | H | H | H | H | H | $Kp_{0,067\ mbar}$ |
| 2 | $CH_2Cl$ | $CH_3$ | $CH_3$ | H | H | $CH_3$ | H | H | 90 °C |
| 3 | $CHCl_2$ | $CH_3$ | $CH_3$ | H | H | H | H | H | Fp: 108 °C |
| 4 | $CHCl_2$ | $CH_3$ | $CH_3$ | H | H | H | $CH_3$ | H | Öl |
| 5 | $CHCl_2$ | $CH_3$ | $CH_3$ | H | H | H | $C_2H_5$ | H | 1,5152 |
| 6 | $CHCl_2$ | $CH_3$ | $CH_3$ | H | H | H | $n-C_3H_7$ | H | 1,5010 |
| 7 | $CHCl_2$ | $CH_3$ | $CH_3$ | H | H | $CH_3$ | H | H | Fp: 56 °C |
| 8 | $CHCl_2$ | $CH_3$ | $CH_3$ | H | H | $CH_3$ | $CH_3$ | H | 1,4918 |
| 9 | $CHCl_2$ | $CH_3$ | $CH_3$ | H | H | $CH_3$ | $C_2H_5$ | H | 1,4949 |
| 10 | $CHCl_2$ | $CH_3$ | $CH_3$ | H | H | $CH_3$ | $n-C_3H_7$ | H | 1,4915 |
| 11 | $CHCl_2$ | $CH_3$ | $CH_3$ | H | H | $CH_3$ | $i-C_3H_7$ | H | 1,4945 |
| 12 | $CHCl_2$ | H | H | $CH_3$ | $CH_3$ | H | H | H | Fp: 64 °C |
| 13 | $CHCl_2$ | H | H | $CH_3$ | $CH_3$ | H | $CH_3$ | H | Fp: 80 °C |
| 14 | $CHCl_2$ | H | H | $CH_3$ | $CH_3$ | H | $C_2H_5$ | H | |
| 15 | $CHCl_2$ | H | H | $CH_3$ | $CH_3$ | H | $i-C_3H_7$ | H | Fp: 84 °C |
| 16 | $CHCl_2$ | $CH_3$ | $CH_3$ | H | H | $CH_3$ | $CH-nC_4H_9$<br>$C_2H_5$ | H | 1,4849 |
| 17 | $CHCl_2$ | $CH_3$ | $CH_3$ | H | H | $CH_3$ | H | $CH(OCH_3)_2$ | Öl |
| 18 | $CHCl_2$ | $CH_3$ | $CH_3$ | H | H | $CH_3$ | $CH_3$ | $CH_3OCH_2$ | Öl |
| 19 | $CHCl_2$ | $CH_3$ | $CH_3$ | H | H | $CH_3$ | – | $(CH_2)_4-$ | Öl |
| 20 | $CHCl_2$ | $CH_3$ | $CH_3$ | H | H | $CH_3$ | – | $(CH_2)_5-$ | Öl |
| 21 | $CCl_3$ | $CH_3$ | $CH_3$ | H | H | H | H | H | Fp: 103 °C |

Die für die Herstellung der neuen Tetrahydro-1,3-oxazine notwendigen Ausgangsverbindungen der Formel III sind zum Teil aus Rec. trav. chim. P.B.78, 315 (1959) und J. Org. Chem. 38, 36 (1973) bekannt. Sie lassen sich nach bekannten Verfahren (Tetrahedron 30, 3315 (1974); Rec. trav. Chim. P.B. 78, 315 (1959)) entsprechend folgender Reaktionsgleichung herstellen:

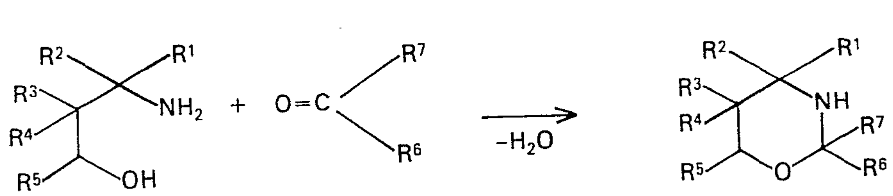


III

Das folgende Beispiel erläutert ihre Herstellung. Gewichtsteile verhalten sich hierbei zu Volumenteilen wie Kilogramm zu Liter.

Beispiel 2

In einem Rührkolben werden 1170 Gewichtsteile 2-Methyl-2-aminopentanol-(4) vorgelegt. Unter äusserer Kühlung lässt man innerhalb von etwa 40 Minuten bei 15-20 °C 440 Gewichtsteile Acetaldehyd zulaufen. Die Reaktionsmischung wird 15 Minuten lang bei 20 °C nachgerührt. Nach Zugabe von 600 Volumenteilen Toluol werden innerhalb von 2 Stunden durch Azeotropdestillation 182 Gewichtsteile Wasser abgetrennt. Das Reaktionsprodukt wird fraktioniert destilliert. Nach Abzug des Lösungsmittels erhält man 1372 Gewichtsteile 2,4,4,6-Tetramethyltetrahydro-1,3-oxazin mit einer gas-chromatographischen Reinheit von 98%, entsprechend einer Ausbeute von 94% d.Th., bezogen auf eingesetztes 2-Methyl-2-amino-pentanol-(4).
Siedepunkt bei 13 mbar: 53-54 °C.
$n_D^{20}$: 1,4340.

Entsprechend werden folgende Verbindungen der Formel III hergestellt:

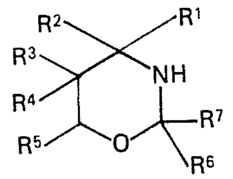

| $R^1$ | $R^2$ | $R^3$ | $R^4$ | $R^5$ | $R^6$ | $R^7$ | $n_D^{20}$ | Kp(mbar) | [°C] |
|---|---|---|---|---|---|---|---|---|---|
| $CH_3$ | $CH_3$ | H | H | H | H | H | 1,4290 | $Kp_{153}$ | 87 |
| $CH_3$ | $CH_3$ | H | H | H | H | $CH_3$ | 1,4372 | $Kp_{20}$ | 42-43 |
| $CH_3$ | $CH_3$ | H | H | H | H | $C_2H_5$ | 1,4392 | $Kp_{26,6}$ | 48-49 |
| $CH_3$ | $CH_3$ | H | H | H | H | $nC_3H_7$ | 1,4371 | $Kp_{26,6}$ | 69-70 |
| $CH_3$ | $CH_3$ | H | H | H | H | $iC_3H_7$ | 1,4391 | $Kp_{26,6}$ | 68-69 |
| $CH_3$ | $CH_3$ | H | H | H | H | $CH<^{C_4H_9}_{C_2H_5}$ | 1,4442 | $Kp_{0,27}$ | 54-55 |
| $CH_3$ | $CH_3$ | H | H | $CH_3$ | H | H | 1,4394 | $Kp_{13,3}$ | 47-48 |
| $CH_3$ | $CH_3$ | H | H | $CH_3$ | H | $CH_3$ | 1,4340 | $Kp_{13,3}$ | 53-54 |
| $CH_3$ | $CH_3$ | H | H | $CH_3$ | H | $C_2H_5$ | 1,4345 | $Kp_{13,3}$ | 58-59 |
| $CH_3$ | $CH_3$ | H | H | $CH_3$ | H | $nC_3H_7$ | 1,4380 | $Kp_{0,27}$ | 41-43 |
| $CH_3$ | $CH_3$ | H | H | $CH_3$ | H | $iC_3H_7$ | 1,4341 | $Kp_{13,3}$ | 65-67 |
| $CH_3$ | $CH_3$ | H | H | $CH_3$ | H | $CH<^{C_4H_9}_{C_2H_5}$ | 1,4451 | $Kp_{0,27}$ | 54-55 |
| $CH_3$ | $CH_3$ | H | H | H | $CH_3$ | $C_2H_5$ | 1,4451 | $Kp_{20}$ | 59-60 |
| $CH_3$ | $CH_3$ | H | H | $CH_3$ | $CH_3$ | $CH_3OCH_2$ | 1,4415 | $Kp_{26,6}$ | 96 |
| $CH_3$ | $CH_3$ | H | H | H | - | $(CH_2)_4-$ | 1,4689 | $Kp_{26,6}$ | 97-98 |
| $CH_3$ | $CH_3$ | H | H | $CH_3$ | - | $(CH_2)_4-$ | 1,4651 | $Kp_{0,27}$ | 47-48 |
| $CH_3$ | $CH_3$ | H | H | $CH_3$ | - | $(CH_2)_5-$ | 1,4690 | $Kp_{0,27}$ | 46-47 |

Herbizide Wirkstoffe und antagonistisch wirkende Verbindungen können gemeinsam oder getrennt vor oder nach der Saat in den Boden eingearbeitet werden. Bei den Acetaniliden ist das gebräuchlichste Anwendungsverfahren die Ausbringung auf die Erdoberfläche unmittelbar nach Ablage der Samen oder in der Zeit zwischen Einsaat und Auflaufen der jungen Pflanzen. Auch eine Behandlung während des Auflaufens ist möglich. In jedem Fall kann das antagonistisch wirkende Mittel gleichzeitig mit dem herbiziden Wirkstoff ausgebracht werden. Auch eine getrennte Ausbringung, wobei der Antagonist zuerst und anschliessend der herbizide Wirkstoff oder umgekehrt auf das Feld gebracht werden, ist möglich, sofern zwischen Ausbringung beider Substanzen nicht soviel Zeit verstreicht, dass der herbizide Wirkstoff bereits Schaden an den Kulturpflanzen anrichtet. Wirkstoff und Antagonist können hierbei als Spritzmittel in suspendierbarer, emulgierbarer oder löslicher Form oder als Granulate getrennt oder gemeinsam formuliert vorliegen. Denkbar ist auch eine Behandlung der Kulturpflanzensamen mit dem Antagonisten vor der Aussaat. Der herbizide Wirkstoff wird dann allein in der üblichen Weise appliziert.

Für das gleiche herbizide Acetanilid werden unterschiedliche Mengen einer antagonistisch wirkenden Verbindung benötigt, wenn das Acetanilid in verschiedenen Kulturen eingesetzt wird. Die Mengenverhältnisse, in denen Acetanilid und Tetrahydro-1,3-oxazin eingesetzt werden, sind in breiten Bereichen variabel. Sie sind abhängig von der Struktur des Acetanilids, des Tetrahydro-1,3-oxazins und der jeweiligen Kultur. Geeignete Anteilsverhältnisse herbizider Wirkstoff:antagonistisch wirkender Verbindung liegen zwischen 1:2 bis 1:0,05 Gewichtsteile.

Die neuen herbiziden Mittel können neben Acetanilid und Tetrahydro-1,3-oxazin weitere herbizide oder wachstumsregulierende Wirkstoffe anderer chemischer Sturktur, beispielsweise
2-Chlor-4-äthylamino-6-isopropylamino-1,3,5-triazin, enthalten, wobei der antagonistische Effekt erhalten bleibt.

Die erfindungsgemässen Mittel bzw. bei getrennter Ausbringung die herbiziden Wirkstoffe oder das Antidot werden beispielsweise in Form von direkt versprühbaren Lösungen, Pulvern, Suspensionen, auch hochprozentige wässrige, ölige oder sonstige Suspensionen, oder Dispersionen, Emulsionen, Öldispersionen, Pasten, Stäubemitteln, Streumitteln oder Granulaten durch Versprühen, Vernebeln, Verstäuben, Verstreuen oder Giessen angewendet. Die Anwendungsformen richten sich ganz nach den Verwendungszwecken; sie sollten in jedem Fall möglichst die feinste Verteilung der erfindungsgemässen Mittel bzw. der Einzelkomponenten gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate, zum Beispiel Methanol, Äthanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wässrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht: Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol, Octylphenol, Nonylphenol, Alkylphenolpolyglykoläther, Tributylphenylpolyglykoläther, Alkalarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreise, Talkum, Bolus, Löss, Ton, Dolomit, Diateomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nusschalenmehl, Cellulosepulver und andere feste Trägerstoffe.

Die Formulierungen enthalten zwischen 0,1 und 95 Gewichtsprozent an herbizidem Wirkstoff und Antidot, vorzugsweise zwischen 0,5 und 90 Gewichtsprozent. Die Aufwandmengen an herbizidem Wirkstoff betragen 0,2 bis 5 kg/ha. Diese Menge an herbizidem Wirkstoff wird, gemeinsam oder getrennt, mit einer solchen Menge an Antidot ausgebracht, das das Anteilsverhältnis herbizider Wirkstoff:antagonistischer Verbindung 1:2 bis 1:0,05 Gewichtsteile beträgt.

Beispiele für Formulierungen sind:

I. 40 Gewichtsteile der Mischung aus vier Gewichtsteilen 2-Chlor-2′,6′-dimethyl-N-(pyrazol-1-yl-methyl)-acetanilid und einem Gewichtsteil N-Dichloracetyl-4,4-dimethyl-tetrahydro-1,3-oxazin werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wässrige Dispersion. Durch Verdünnen mit 100 000 Gewichtsteilen Wasser erhält man eine wässrige Dispersion, die 0,04 Gewichtsprozent Wirkstoff enthält.

II. 3 Gewichtsteile der Mischung aus einem Gewichtsteil 2-Chlor-2′,6′-dimethyl-N-(pyrazol-1-yl-methyl)-acetanilid und einem Gewichtsteil N-Dichloracetyl-4,4,6-trimethyl-tetrahydro-1,3-oxazin werden mit 97 Gewichtsteilen feinteiligem Kaolin innig vermischt. Man erhält auf diese Weise ein Stäubemittel, das 3 Gewichtsprozent des Wirkstoffs enthält.

III. 30 Gewichtsteile der Mischung aus einem Gewichtsteil 2-Chlor-2′-methyl-6′-äthyl-N-(1,2,4-triazol-1-yl-methyl)-acetanilid und zwei Gewichtsteilen N-Dichloracetyl-2-äthyl-4,4,6-trimethyl-tetrahydro-1,3-oxazin werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 20 Teile der Mischung aus 8 Gewichtsteilen 2-Chlor-2′-methyl-6′-äthyl-N-(pyrazol-1-yl-methyl)-acetanilid und einem Gewichtsteil N-Dichloracetyl-2-n-propyl-4,4,6-trimethyl-1,3-tetrahydro-1,3-oxazin werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

V. 20 Gewichtsteile der Mischung aus zehn Gewichtsteilen 2-Chlor-2′,6′-dimethyl-N-(4,5-dichlorimidazol-1-yl-methyl)-acetanilid und einem Gewichtsteil N-Dichloracetyl-4,4-dimethyl-tetrahydro-1,3-oxazin werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingiessen und feines Verteilen der Lösung in 100000 Gewichtsteilen Wasser erhält man eine wässrige Dispersion, die 0,02 Gewichtsprozent des Wirkstoffs enthält.

Der Einfluss verschiedener Vertreter der erfindungsgemässen herbiziden Mittel auf das Wachstum von erwünschten und unerwünschten Pflanzen im Vergleich zu dem herbizider Mittel aus den gleichen herbiziden Wirkstoffen und einer antagonistisch wirkenden, bereits bekannten Verbindung chemisch ähnlicher Struktur wird durch die folgenden biologischen Beispiele belegt. Die Versuche zeigen, dass die Verträglichkeit der herbiziden Acetanilide durch kombinierte Anwendung mit den neuen Tetrahydro-1,3-oxazinen bei gleichbleibender herbizider Wirkung verbessert wird.

Die Versuchsserien wurden im Gewächshaus und im Freiland durchgeführt.

I. Gewächshausversuche

Plastikpikierkisten von 51 cm Länge, 32 cm Breite und 6 cm Höhe wurden mit lehmigem Sand von pH 6 und etwa 1,5% Humus gefüllt. In dieses Substrat wurde Mais (Zea mays) oder Weizen (Triticum aestivum) in Reihen flach eingesät. Ausserdem wurde Echinochloa crus galli und Alopecurus myosuroides als unerwünschte Pflanzen breitwürfig hinzugegeben. Der nicht sterilisierte Boden enthielt zusätzlich lebensfähige Unkrautsamen, welche zur Population unerwünschter Pflanzen beitrugen. Dadurch wurde ein mit Kulturpflanzen bestellter und mit Unkräutern verseuchter Acker simuliert.

Wirkstoffe und Antagonisten wurden sowohl einzeln als auch in den beschriebenen Mischungen aufgebracht. Dazu verteilte man sie, emulgiert oder suspendiert in Wasser als Trägermedium, und spritzte sie mittels fein verteilender Düsen unmittelbar nach der Saat und vor Auflaufen der Testpflanzen auf die Erdoberfläche. In einigen Fällen wurden die Mittel auch vor Einsaat der Kulturpflanzen in den Boden eingemischt. Nach Einsaat und Behandlung wurden die Kisten beregnet und bis nach dem Auflaufen der Pflanzen mit durchsichtigen Plastikhauben abgedeckt. Durch diese Massnahmen wurde ein gleichmässiges Keimen und Anwachsen der Pflanzen garantiert. Die Aufstellung erfolgte in einem Temperaturbereich von durchschnittlich 18 bis 30 °C.

Die so angelegten Gewächshausversuche wurden beobachtet, bis der Mais 3 bis 5 Blätter entwickelt hatte. Nach diesem Stadium waren bei den herbiziden Mitteln keine Schädigungen mehr zu erwarten, was auch in den Freilandversuchen bestätigt wurde. Die Bewertung der Mitteleinwirkung geschah nach einer Skala von 0 bis 100. Hierbei bedeutete 0 normaler Aufgang und Entwicklung der Pflanzen, bezogen auf die unbehandelte Kontrolle. 100 entsprach einem völligen Ausbleiben der Keimung bzw. Absterben der Pflanzen. Es musste dabei berücksichtigt werden, dass z.B. bei Mais auch unter völlig normalen Verhältnissen und ohne jede chemische Behandlung vereinzelt verkrüppelte oder gehemmte Pflanzen vorkommen.

II. Freilandversuche

Die Freilandversuche wurden auf Kleinparzellen auf Standorten mit lehmigem Sand und Lehm von pH 5 bis 6 und 1 bis 1,5% Humusgehalt angelegt. Die Vorauflaufbehandlungen erfolgten jeweils unmittelbar bis spätestens 3 Tage nach der Saat der Kulturpflanzen. Die Unkrautflora verschiedenster Artenzusammensetzung war natürlich. Allerdings wurden nur die dominierenden Vertreter in die Tabellen aufgenommen. Wirkstoffe und Antagonisten sowie deren Kombinationen wurden in Wasser als Träger- und Verteilermedium emulgiert oder suspendiert und mittels einer motorgetriebenen, auf einen Traktor montierten Parzellenspritze ausgebracht. Bei Mangel an natürlichen Niederschlägen wurde zusätzlich beregnet, um ein normales Auflaufen von Kultur und Unkräutern zu gewährleisten. Alle Versuche liefen über mehrere Monate, so dass die Entwicklung der Kulturpflanze bis zur Samenbildung beobachtet werden konnte. Die Bewertung der Mittelwirkung wurde ebenfalls nach der Skala von 0 bis 100 vorgenommen.

Ergebnis

Bedingt durch die flache Einsaat der Kulturpflanzen und die für die herbizide Aktivität günstigeren Bedingungen traten die durch die herbiziden Wirkstoffe verursachten Schäden im Gewächshaus viel stärker zutage als im Freiland. Die Prüfung der antagonistisch wirkenden Verbindungen war folglich im Gewächshaus härter als im Freiland.

Im einzelnen zeigen die tabellarisch aufgeführten Versuchsergebnisse:

Die neuen antagonistisch wirkenden Tetrahydro-1,3-oxazine haben, für sich allein ausgebracht, keinen oder einen kaum sichtbaren Einfluss auf Keimung und Wachstum von Kulturpflanzen oder unerwünschten Pflanzen. Das zeigt sich auch bei Aufwandmengen, welche beträchtlich über denjenigen liegen, welche für antagonistische Effekte Voraussetzung sind.

Die neuen Verbindungen reduzieren jedoch die Phytotoxizität der herbiziden Acetanilide der Formel I gegenüber Kulturpflanzen, wie Mais und Getreide, beachtlich und eleminieren sie zum Teil völlig. Es zeigt sich, dass bei herbiziden Verbindungen, die gegenüber Kulturpflanzen weniger aggressiv sind, auch der Zusatz kleinerer Mengen antagonistisch wirkender Substanzen oder solcher mit geringerer antagonistischer Aktivität ausreichen.

Tabelle 1 – Liste der Pflanzennamen

| Botanischer Name | Abkürzung in Tabellen | Deutscher Name | Englischer Name |
|---|---|---|---|
| Alopecurus myosuroides | | Ackerfuchsschwanz | slender foxtail |
| Chenopodium album | | Weisser Gänsefuss | lambsquarters |
| Echinochloa crus galli | Echinochloa c.g. | Hühnerhirse | barnyardgrass |
| Galinsoga spp. | | Franzosenkraut | galant soldier |
| Hordeum vulgare | | Gerste | barley |
| Matricaria Anthemis spp. | | Kamillearten | chamomile |
| Triticum aestivum | | Weizen | wheat |
| Zea mays | | Mais | Indian corn |

Tabelle 2 – Liste der in den biologischen Beispielen aufgeführten herbiziden Acetanilide

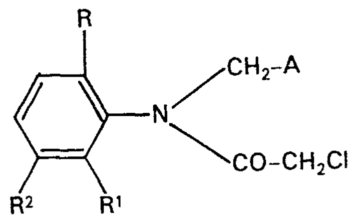

| Bezeichnung | A | R | $R^1$ | $R^2$ | Fp [°C] |
|---|---|---|---|---|---|
| A | 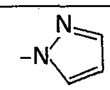 | $CH_3$ | $CH_3$ | H | 81 |
| B | 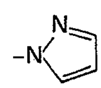 | $C_2H_5$ | $CH_3$ | H | 56 |
| C | 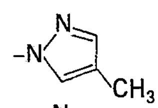 | $CH_3$ | $CH_3$ | H | 102 |
| D | 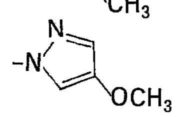 | $C_2H_5$ | $CH_3$ | H | 94 |
| E | 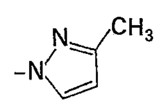 | $C_2H_5$ | $CH_3$ | H | Öl |
| F | 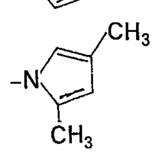 | $CH_3$ | $CH_3$ | H | 126 |
| G | 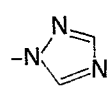 | $CH_3$ | $CH_3$ | H | 120 |
| H | 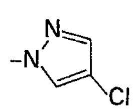 | $CH_3$ | $CH_3$ | H | 104 |
| I | 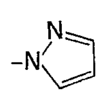 | $CH_3$ | $CH_3$ | $CH_3$ | 92 |

Tabelle 3
Liste der in den biologischen Beispielen aufgeführten antagonistisch wirkenden Tetrahydro-1,3-oxazine

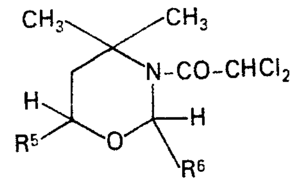

| Nr. | $R^5$ | $R^6$ |
|---|---|---|
| 3 | H | H |
| 7 | $CH_3$ | H |
| 8 | $CH_3$ | $CH_3$ |
| 9 | $CH_3$ | $C_2H_5$ |
| 10 | $CH_3$ | $n-C_3H_7$ |
| 11 | $CH_3$ | $i-C_3H_7$ |
| 16 | $CH_3$ | $-CH(C_2H_5)-n-C_4H_9$ |
| V (bekannt) | 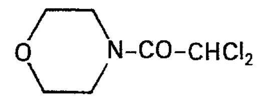 | |

Tabelle 4
Reduktion von Schäden an Mais durch 2-Chlor-2',6'-dimethyl-N-(pyrazol-1-yl-methyl)-acetanilid bei Zugabe antagonistisch wirkender Tetrahydro-1,3-oxazine bei Vorauflaufanwendung im Gewächshaus.

| Herbizider Wirkstoff | Antagonistisch wirkende Verbindung | Aufwandmenge [kg/ha a.S.] | Testpflanzen und % Schädigung Kulturpflanze Zea mays | unerwünschte Pflanzen Alopecurus myosuroides | Echinochloa c.g. |
|---|---|---|---|---|---|
| A | – | 1,0 | 57 | 100 | 100 |
| | | 2,0 | 73 | 100 | 100 |
| A | 3 | 1+0,125 | 8 | 99 | 99 |
| | | 1+1,0 | 5 | 100 | 100 |
| | | 1+2,0 | 5 | 100 | 100 |
| | | 2+0,25 | 15 | 100 | 100 |
| | | 2+0,5 | 10 | 100 | 100 |
| A | 7 | 1+0,125 | 0 | 98 | 98 |
| | | 1+0,25 | 0 | 100 | 100 |
| | | 1+2,0 | 5 | 100 | 100 |
| | | 2+0,5 | 10 | 100 | 100 |
| | | 2+2,0 | 10 | 100 | 100 |
| A | 8 | 1+0,25 | 15 | 100 | 100 |
| | | 1+2,0 | 15 | 100 | 100 |
| A | 9 | 1+0,25 | 25 | 100 | 100 |
| | | 1+2,0 | 25 | 100 | 100 |
| A | 10 | 1+2 | 25 | 100 | 100 |
| A | 11 | 1+0,25 | 20 | 100 | 100 |
| A | 16 | 1+0,25 | 20 | 100 | 100 |
| – | 3 | 4,0 | 2,5 | 0 | 0 |
| – | 7 | 4,0 | 0 | 0 | 0 |
| – | 8 | 4,0 | 0 | 0 | 0 |
| – | 9 | 4,0 | 0 | 0 | 0 |
| – | 10 | 4,0 | 0 | 0 | 0 |
| – | 11 | 4,0 | 0 | 0 | 0 |
| – | 16 | 4,0 | 0 | 0 | 0 |

0 = normaler Aufgang, keine Schädigung
10 = Pflanzen nicht aufgelaufen oder abgestorben

Tabelle 5

Verbesserung der Maisverträglichkeit von herbiziden Acetaniliden durch antagonistisch wirkende Tetrahydro-1,3-oxazine bei Vorauflaufanwendung im Gewächshaus.

| Herbizider Wirkstoff | Antagonistisch wirkende Verbindung | Aufwandmengen [kg/ha a.S.] | Testpflanzen und % Schädigung Kulturpflanze Zea mays | unerwünschte Pflanze Echinochloa c.g. |
|---|---|---|---|---|
| A | – | 1,0 | 88 | 100 |
| | | 2,0 | 90 | 100 |
| – | 3 | 4,0 | 10 | 0 |
| A | 3 | 1,0+0,125 | 10 | 100 |
| | | 1,0+0,5 | 0 | 100 |
| | | 1,0+2,0 | 5 | 100 |
| B | – | 1,0 | 70 | 100 |
| | | 2,0 | 88 | 100 |
| B | 3 | 1,0+0,25 | 0 | 100 |
| | | 2,0+0,5 | 5 | 100 |
| | | 2,0+2,0 | 5 | 100 |
| C | – | 1,0 | 30 | 100 |
| | | 2,0 | 70 | 100 |
| C | 3 | 1,0+0,25 | 0 | 100 |
| | | 1,0+1,0 | 0 | 100 |
| | | 2,0+0,25 | 0 | 100 |
| | | 2,0+2,0 | 5 | 100 |
| D | – | 1,0 | 10 | 100 |
| | | 2,0 | 65 | 100 |
| D | 3 | 1,0+0,25 | 0 | 100 |
| | | 1+1 | 0 | 100 |
| | | 2,0+0,5 | 5 | 100 |
| | | 2,0+2,0 | 0 | 100 |
| E | – | 1,0 | 70 | 100 |
| | | 2,0 | 80 | 100 |
| E | 3 | 1,0+0,25 | 0 | 100 |
| | | 2,0+0,5 | 0 | 100 |
| F | – | 1,0 | 70 | 100 |
| | | 2,0 | 85 | 100 |
| F | 3 | 1,0+1,0 | 10 | 100 |
| | | 1,0+0,25 | 20 | 100 |
| | | 2,0+2,0 | 10 | 100 |
| G | – | 1,0 | 30 | 99 |
| | | 2,0 | 62 | 100 |
| G | 3 | 1,0+0,125 | 5 | 98 |
| | | 1,0+1,0 | 5 | 98 |
| | | 2,0+0,5 | 10 | 98 |
| H | – | 2,0 | 8 | 96 |
| H | 3 | 2,0+0,5 | 5 | 95 |
| I | – | 1,0 | 75 | 98 |
| I | 3 | 1,0+1,0 | 0 | 96 |
| | | 1,0+0,125 | 5 | 95 |

0 = normaler Aufgang, keine Schädigung
10 = Pflanzen nicht aufgelaufen oder abgestorben

Tabelle 6
Verbesserung der Maisverträglichkeit von 2-Chlor-2',6'-dimethyl-N-(pyrazol-1-yl-methyl)-acetanilid durch Zumischung von antagonistisch wirkendes N-Dichloracetyl-4,4-dimethyl-tetrahydro-1,3-oxazin)

| Herbizider Wirkstoff | Antagonistische Verbindungen | Aufwandmengen [kg/ha a.S.] | Testpflanzen und % Schädigung Kultur Zea mays | unerwünschte Pflanzen Cheno-podium album | Matricaria/ Anthemis spp. | Galinsoga spp. |
|---|---|---|---|---|---|---|
| A | - | 1,0 | 4 | 95 | 100 | 98 |
| | | 2,0 | 15 | 100 | 100 | 100 |
| | | 3,0 | 14 | 100 | 100 | - |
| A | 3 | 1+0,125 | 0 | - | - | - |
| | | 1+0,5 | 2 | 98 | 100 | 100 |
| | | 2+0,25 | 2,5 | - | 100 | - |
| | | 2+1,0 | 5 | 100 | 100 | 100 |
| | | 3+1,0 | 5 | 100 | 100 | - |

0 = normaler Aufgang, keine Schädigung
100 = Pflanzen nicht aufgelaufen oder abgestorben

Tabelle 7
Steigerung der Getreideverträglichkeit von herbiziden Acetaniliden durch N-Dichloracetyl-4,4-dimethyl-tetrahydro-1,3-oxazin bei Vorauflaufanwendung im Gewächshaus

| Herbizider Wirkstoff | Antagonistische Verbindungen | Aufwandmengen [kg/ha a.S.] | Testpflanzen und % Schädigung Kulturpflanzen Hordeum vulgare | Triticum aestivum | unerwünschte Pflanze Alopecurus myosuroides |
|---|---|---|---|---|---|
| H | - | 1,0 | 28 | 22 | 93 |
| | | 2,0 | 60 | 45 | 98 |
| H | 3 | 2,0+0,5 | 20 | 2 | 94 |
| | | 1,0+1,0 | 2 | 5 | 82 |
| | | 1,0+0,125 | 15 | 5 | 92 |
| | | 1,0+2,0 | 5 | 5 | 95 |
| D | - | 1,0 | 35 | 35 | 94 |
| D | 3 | 1,0+0,5 | 32 | 15 | 98 |
| | | 1,0+2,0 | 5 | 5 | 94 |

Tabelle 8
Vergleich der antagonistischen Wirkung eines erfindungsgemässen Tetrahydro-1,3-oxazins mit einer bekannten antagonistisch wirkenden Verbindung bei Vorauflaufanwendung im Gewächshaus

| Herbizider Wirkstoff | Antagonistisch wirkende Verbindung | Aufwandmengen [kg/ha a.S.] | Testpflanzen und % Schädigung Kulturpflanze Zea mays | unerwünschte Pflanze Echinochloa c.g. |
|---|---|---|---|---|
| A | - | 1,0 | 74 | 99 |
| | | 2,0 | 82 | - |
| | | 3,0 | 84 | - |
| - | 3 | 4,0 | 1 | 0 |
| - | V (bekannt) | 4,0 | 1 | 2 |
| A | 3 | 1,0+0,125 | 18 | 99 |
| | | 2,0+0,5 | 26 | - |
| | | 3,0+0,5 | 42 | - |
| A | V (bekannt) | 1,0+0,125 | 42 | 99 |
| | | 2,0+0,5 | 48 | - |
| | | 3,0+0,5 | 70 | - |

0 = normaler Aufgang, keine Schädigung
100 = Pflanzen nicht aufgelaufen oder abgestorben

**Patentansprüche**
**BE, CH, DE, FR, GB, JT, LU, NL, SE**

1. Tetrahydro-1,3-oxazine der Formel II

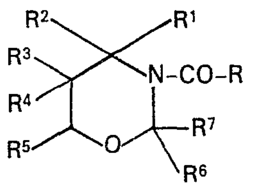

II

in der
R einen unverzweigten oder verzweigten Halogenalkylrest mit bis zu 3 Kohlenstoffatomen,
$R^1$, $R^2$, $R^3$, $R^4$ und $R^5$ gleich oder verschieden sind und Wasserstoff oder einen unverzweigten oder verzweigten Alkylrest mit bis zu 3 Kohlenstoffatomen,
$R^6$ Wasserstoff oder einen unverzweigten oder verzweigten Alkylrest mit bis zu 8 Kohlenstoffatomen,
$R^7$ Wasserstoff, einen unverzweigten oder verzweigten Alkylrest mit bis zu 8 Kohlenstoffatomen, einen Alkoxyalkylrest mit bis zu 6 Kohlenstoffatomen oder einen Dialkoxy-alkylrest mit bis zu 8 Kohlenstoffatomen bedeuten und
$R^6$ und $R^7$ zusammen eine Methylenkette mit 4 oder 5 Kohlenstoffatomen bilden können.

2. N-Dichloracetyl-4,4-dimethyl-tetrahydro-1,3-oxazin nach Anspruch 1.

3. N-Dichloracetyl-4,4,6-trimethyl-tetrahydro-1,3-oxazin nach Anspruch 1.

4. Herbizides Mittel, gekennzeichnet durch einen Gehalt an mindestens einem substituierten Acetanilid der Formel I

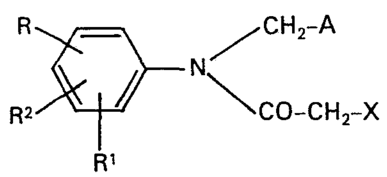

I

in der
R Wasserstoff, einen unverzweigten oder verzweigten Alkyl- oder Alkoxyrest mit bis zu 5 Kohlenstoffatomen,
$R^1$ Wasserstoff, Halogen, einen unverzweigten oder verzweigten Alkyl- oder Alkoxyrest mit bis zu 5 Kohlenstoffatomen,
$R^2$ Wasserstoff, Halogen, einen unverzweigten oder verzweigten Alkyl- oder Alkoxyrest mit bis zu 5 Kohlenstoffatomen,
R zusammen mit $R^2$ eine orthoständig verknüpfte, gegebenenfalls durch unverzweigte oder verzweigte Alkylgruppen mit bis zu 4 Kohlenstoffatomen substituierte Alkylenkette mit bis zu 6 Kohlenstoffatomen,
X Chlor oder Brom und
A ein über ein Ringstickstoffatom gebundenes Azol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy, Carbalkoxy mit bis zu 4 Kohlenstoffatomen in der Alkoxygruppe oder Alkanoylreste mit bis zu 4 Kohlenstoffatomen substutiert sein kann, bedeutet, wobei A auch für Salze der 2 oder 3 Stickstoffatome enthaltenden Azole stehen kann,
als herbizidem Wirkstoff und
mindestens einem Tetrahydro-1,3-oxazin nach Anspruch 1 als antagonistischem Mittel.

5. Herbizides Mittel nach Anspruch 4, dadurch gekennzeichnet, dass es 2-Chlor-2′,6′-dimethyl-N-(pyrazol-1-yl-methyl)-acetanilid als herbiziden Wirkstoff und N-Dichloracetyl-4,4-dimethyl-tetrahydro-1,3-oxazin als antagonistisches Mittel enthält.

6. Herbizides Mittel nach Anspruch 4, dadurch gekenzeichnet, dass es 2-Chlor-2′,6′-dimethyl-N-(pyrazol-1-yl-methyl)-N-(pyrazol-1-yl-methyl)-acetanilid als herbizides Mittel und N-Dichloracetyl-4,4,6-trimethyl-tetrahydro-1,3-oxazin als antagonistisches Mittel enthält.

7. Herbizides Mittel nach Anspruch 4, dadurch gekennzeichnet, dass das Anteilsverhältnis Acetanilid:Tetrahydro-1,3-oxazin bei gemeinsamer oder getrennter Ausbringung 1:2 bis 1:0,05 Gewichtsteile beträgt.

8. Verfahren zur selektiven Bekämpfung von unerwünschtem Pflanzenwuchs, dadurch gekennzeichnet, dass man substituierte Acetanilide der Formel I nach Anspruch 4 und Tetrahydro-1,3-oxazine der Formel II nach Anspruch 1 vor, bei oder nach der Aussaat der Kulturpflanzen, vor oder während des Auflaufens der Kulturpflanzen gleichzeitig oder nacheinander in beliebiger Reihenfolge ausbringt.

9. Verfahren zur selektiven Bekämpfung von unerwünschtem Pflanzenwuchs mit substituierten Acetaniliden der Formel I nach Anspruch 4, dadurch gekennzeichnet, das man das Saatgut der Kulturpflanzen mit einem oder mehreren der Tetrahydro-1,3-oxazine der Formel II nach Anspruch 1 behandelt.

10. Verfahren nach Anspruch 8 oder 9, dadurch gekennzeichnet, dass die Kulturpflanze Mais oder Getreide ist.

**Patentansprüche AT**

1. Herbizides Mittel, gekennzeichnet durch einen Gehalt an mindestens einem substituierten Acetanilid der Formel I

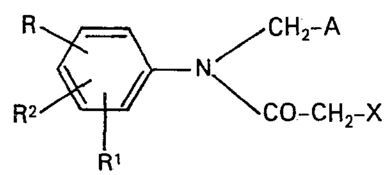

I

in der
R Wasserstoff, einen unverzweigten oder verzweigten Alkyl- oder Alkoxyrest mit bis zu 5 Kohlenstoffatomen,
$R^1$ Wasserstoff, Halogen, einen unverzweigten oder verzweigten Alkyl- oder Alkoxyrest mit bis zu 5 Kohlenstoffatomen,

$R^2$ Wasserstoff, Halogen, einen unverzweigten oder verzweigten Alkyl- oder Alkoxyrest mit bis zu 5 Kohlenstoffatomen,
R zusammen mit $R^2$ eine orthoständig verknüpfte, gegebenenfalls durch unverzweigte oder verzweigte Alkylgruppen mit bis zu 4 Kohlenstoffatomen substituierte Alkylenkette mit bis zu 6 Kohlenstoffatomen,
X Chlor oder Brom und
A ein über ein Ringstickstoffatom gebundenes Azol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy, Carbalkoxy mit bis zu 4 Kohlenstoffatomen in der Alkoxygruppe oder Alkanoylreste mit bis zu 4 Kohlenstoffatomen substituiert sein kann, bedeutet, wobei A auch für Salze der 2 oder 3 Stickstoffatome enthaltenden Azole stehen kann,
als herbizidem Wirkstoff und
mindestens einem Tetrahydro-1,3-oxazin der Formel II

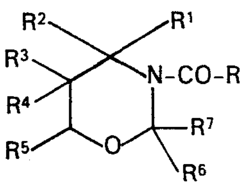
II

in der
R einen unverzweigten oder verzweigten Halogenalkylrest mit bis zu 3 Kohlenstoffatomen,
$R^1$, $R^2$, $R^3$, $R^4$ und $R^5$ gleich oder verschieden sind und Wasserstoff oder einen unverzweigten oder verzweigten Alkylrest mit bis zu 3 Kohlenstoffatomen,
$R^6$ Wasserstoff oder einen unverzweigten oder verzweigten Alkylrest mit bis zu 8 Kohlenstoffatomen,
$R^7$ Wasserstoff, einen unverzweigten oder verzweigten Alkylrest mit bis zu 8 Kohlenstoffatomen, einen Alkoxyalkylrest mit bis zu 6 Kohlenstoffatomen oder einen Dialkoxy-alkylrest mit bis zu 8 Kohlenstoffatomen bedeuten und
$R^6$ und $R^7$ zusammen eine Methylenkette mit 4 oder 5 Kohlenstoffatomen bilden können,
als antagonistischem Mittel.

2. Herbizides Mittel nach Anspruch 1, dadurch gekennzeichnet, dass es N-Dichloracetyl-4,4-dimethyl-tetrahydro-1,3-oxazin als antagonistisches Mittel enthält.

3. Herbizides Mittel nach Anspruch 1, dadurch gekennzeichnet, dass es N-Dichloracetyl-4,4,6-trimethyl-tetrahydro-1,3-oxazin als antagonistisches Mittel enthält.

4. Herbizides Mittel nach Anspruch 1, dadurch gekennzeichnet, dass es 2-Chlor-2',6'-dimethyl-N-(pyrazol-1-yl-methyl)-acetanilid als herbiziden Wirkstoff und N-Dichloracetyl-4,4-dimethyl-tetrahydro-1,3-oxazin als antagonistisches Mittel enthält.

5. Herbizides Mittel nach Anspruch 1, dadurch gekennzeichnet, das es 2-Chlor-2',6'-dimethyl-N-(pyrazol-1-yl-methyl)-N-(pyrazol-1-yl-methyl)-acetanilid als herbizides Mittel und N-Dichloracetyl-4,4,6-trimethyl-tetrahydro-1,3-oxazin als antagonistisches Mittel enthält.

6. Herbizides Mitel nach Anspruch 1, dadurch gekennzeichnet, dass das Anteilsverhältnis Acetanilid:Tetrahydro-1,3-oxazin bei gemeinsamer oder getrennter Ausbringung 1:2 bis 1:0,05 Gewichtsteile beträgt.

**Claims BE, CH, DE, FR, GB, JT, LU, NL, SE**

1. A tetrahydro-1,3-oxazine of the formula II

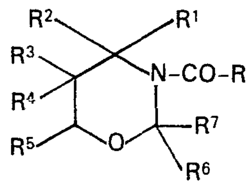
II

where
R denotes linear or branched haloalkyl of up to 3 carbon atoms,
$R^1$, $R^2$, $R^3$ $R^4$ and $R^5$ are identical or different and each denotes hydrogen or linear or branched alkyl of up to 3 carbon atoms,
$R^6$ denotes hydrogen or linear or branched alkyl of up to 8 carbon atoms,
$R^7$ denotes hydrogen, linear or branched alkyl of up to 8 carbon atoms, alkoxyalkyl of up to 6 carbon atoms or dialkoxyalkyl of up to 8 carbon atoms, and
$R^6$ and $R^7$ may together form a methylene chain of 4 or 5 carbon atoms.

2. N-dichloroacetyl-4,4-dimethyltetrahydro-1,3-oxazine as claimed in claim 1.

3. N-dichloroacetyl-4,4,6-trimethyltetrahydro-1,3-oxazine as claimed in claim 1.

4. A herbicidal agent characterized by a content of at least one substituted acetanilide of the formula I

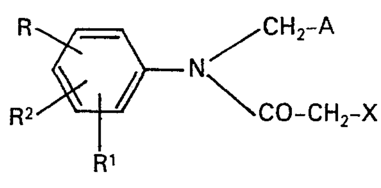
I

where
R denotes hydrogen, linear or branched alkyl or alkoxy of up to 5 carbon atoms,
$R^1$ denotes hydrogen, halogen, or linear or branched alkyl or alkoxy of up to 5 carbon atoms,
$R^2$ denotes hydrogen, halogen, or linear or branched alkyl or alkoxy of up to 5 carbon atoms,
R together with $R^2$ denotes an alkylene chain of up to 6 carbon atoms which is linked in the o-position and may be substituted by linear or branched alkyl of up to 4 carbon atoms,
X denotes chlorine or bromine, and
A denotes azole which is attached via a ring nitrogen atom and may be mono- or polysubstituted by halogen, phenyl, alkyl, alkoxy, alkylthio or

perfluoroalkyl, each of up to 4 carbon atoms, cyano, carboxy, carbalkoxy of up to 4 carbon atoms in the alkoxy, or alkanoyl of up to 4 carbon atoms, or A denotes salts of azoles containing 2 or 3 nitrogen atoms, as herbicidal active ingredient, and of at least one tetrahydro-1,3-oxazine as claimed in claim 1, as antagonistic agent.

5. A herbicidal agent as claimed in claim 4, characterized in that it contains 2-chloro-2',6'-dimethyl-N-(pyrazol-1-yl-methyl)-acetanilide as herbicidal active ingredient and N-dichloroacetyl-4,4-dimethyltetrahydro-1,3-oxazine as antagonistic agent.

6. A herbicidal agent as claimed in claim 4, characterized in that it contains 2-chloro-2',6'-dimethyl-N-(pyrazol-1-yl-methyl)-N-(pyrazol-1-yl-methyl)-acetanilide as herbicidal active ingredient and N-dichloroacetyl-4,4,6-trimethyltetrahydro-1,3-oxazine as antagonistic agent.

7. A herbicidal agent as claimed in claim 4, characterized in that the ratio of acetanilide to tetrahydro-1,3-oxazine, applied separately or together, is from 1:2 to 1:0.05 parts by weight.

8. A process for the selective control of unwanted plants, characterized in that a substituted acetanilide of the formula I as claimed in claim 4 and a tetrahydro-1,3-oxazine of the formula II as claimed in claim 1 are applied, either simultaneously or one after the other in any order, before, during or after sowing of the crop plants or before or during emergence of the crop plants.

9. A process for the selective control of unwanted plants with a substituted acetanilide of the formula I as claimed in claim 4, characterized in that the crop plant seed is treated with one or several tetrahydro-1,3-oxazines of the formula II as claimed in claim 1.

10. A process as claimed in claim 8 or 9, characterized in that the crop plant is Indian corn or a cereal.

**Claims AT**

1. A herbicidal agent characterized by a content of at least one substituted acetanilide of the formula I

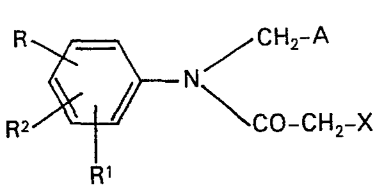

I

where

R denotes hydrogen, linear or branched alkyl or alkoxy of up to 5 carbon atoms,

$R^1$ denotes hydrogen, halogen, or linear or branched alkyl or alkoxy of up to 5 carbon atoms,

$R^2$ denotes hydrogen, halogen, or linear or branched alkyl or alkoxy of up to 5 carbon atoms,

R together with $R^2$ denotes an alkylene chain of up to 6 carbon atoms which is linked in the o-position and may be substituted by linear or branched alkyl of up to 4 carbon atoms,

X denotes chlorine or bromine, and

A denotes azole which is attached via a ring nitrogen atom and may be mono- or polysubstituted by halogen, phenyl, alkyl, alkoxy, alkylthio or perfluoroalkyl, each of up to 4 carbon atoms, cyano, carboxy, carbalkoxy of up to 4 carbon atoms in the alkoxy, or alkanoyl of up to 4 carbon atoms, or A denotes salts of azoles containing 2 or 3 nitrogen atoms,

as herbicidal active ingredient, and of at least one tetrahydro-1,3-oxazine of the formula II

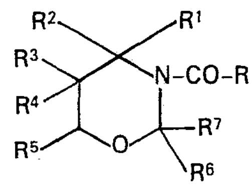

II

where

R denotes linear or branched haloalkyl of up to 3 carbon atoms,

$R^1$, $R^2$, $R^3$, $R^4$ and $R^5$ are identical or different and each denotes hydrogen or linear or branched alkyl of up to 3 carbon atoms,

$R^6$ denotes hydrogen or linear or branched alkyl of up to 8 carbon atoms,

$R^7$ denotes hydrogen, linear or branched alkyl of up to 8 carbon atoms, alkoxyalkyl of up to 6 carbon atoms or dialkoxyalkyl of up to 8 carbon atoms and

$R^6$ and $R^7$ may together form a methylene chain of 4 or 5 carbon atoms, as antagonistic agent.

2. A herbicidal agent as claimed in claim 1, characterized in that it contains N-dichloroacetyl-4,4-dimethyltetrahydro-1,3-oxazine as antagonistic agent.

3. A herbicidal agent as claimed in claim 1, characterized in that it contains N-dichloroacetyl-4,4,6-trimethyltetrahydro-1,3-oxazine as antagonistic agent.

4. A herbicidal agent as claimed in claim 1, characterized in that it contains 2-chloro-2',6'-dimethyl-N-(pyrazol-1-yl-methyl)-acetanilide as herbicidal active ingredient and N-dichloroactyl-4,4-dimethyltetrahydro-1,3-oxazine as antagonistic agent.

5. A herbicidal agent as claimed in claim 1, characterized in that it contains 2-chloro-2',6'-dimethyl-N-(pyrazol-1-yl-methyl)-N-(pyrazol-1-yl-methyl)-acetanilide as herbicidal active ingredient and N-dichloroacetyl-4,4,6-trimethyltetrahydro-1,3-oxazine as antagonistic agent.

6. A herbicidal agent as claimed in claim 1, characterized in that the ratio of acetanilide to tetrahydro-1,3-oxazine, applied separately or together, is from 1:2 to 1:0.05 parts by weight.

**Revendications BE, CH, DE, FR, GB, JT, LU, NL, SE**

1. Tétrahydro-1,3-oxazines de la formule générale

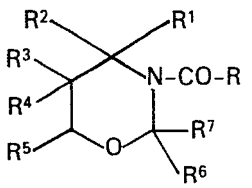

II

dans laquelle
R désigne un radical halo-alcoyle à chaîne droite ou ramifiée en $C_1$ à $C_3$;
$R^1$, $R^2$,$R^3$, $R^4$ et $R^5$, qui peuvent être identiques ou différents, désignent chacun un atome d'hydrogène ou un radical alcoyle à chaîne droite ou ramifiée en $C_1$ à $C_3$;
$R^6$ représente un atome d'hydrogène ou un radical alcoyle à chaîne droite ou ramifiée en $C_1$ à $C_8$;
$R^7$ représente un atome d'hydrogène, un radical alcoyle à chaîne droite ou ramifiée en $C_1$ à $C_8$, un radical alcoxy-alcoyle comprenant jusqu'à 6 atomes de carbone ou un radical dialcoxy-alcoyle comprenant jusqu'à 8 atomes de carbone,
$R^6$ et $R^7$ pouvant former ensemble une chaîne méthylène en $C_4$ ou $C_5$.

2. La N-dichloracétyl-4,4-diméthyl-tétrahydro-1,3-oxazine suivant la revendication 1.

3. La N-dichloracétyl-4,4,6-triméthyl-tétrahydro-1,3-oxazine suivant la revendication 1.

4. Composition herbicide, caractérisée en ce qu'elle contient comme agent herbicide actif au moins un acétanilide substitué de la formule générale

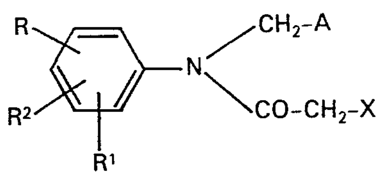

I

dans laquelle
R désigne un atome d'hydrogène ou un radical alcoyle ou alcoxy à chaîne droite ou ramifiée en $C_1$ à $C_5$;
$R^1$ désigne un atome d'hydrogène ou d'halogène ou un radical alcoyle ou alcoxy à chaîne droite ou ramifiée en $C_1$ à $C_5$;
$R^2$ désigne un atome d'hydrogène ou d'halogène ou un radical alcoyle ou alcoxy à chaîne droite ou ramifiée en $C_1$ à $C_5$ et forme avec R une chaîne alcoylène comprenant jusqu'à 6 atomes de carbone, éventuellement substituée par des radicaux alcoyle à chaîne droite ou ramifiée en $C_1$ à $C_4$, à connexion en position ortho;
X représente un atome de chlore ou de brome et
A représente un groupe azole lié par un atome d'azote du noyau et qui peut porter un ou plusieurs substituants choisis parmi les atomes d'halogène et les groupes phényle, alcoyle, alcoxy, alcoyl-thio et perfluoralcoyle comportant jusqu'à 4 atomes de carbone, cyano, carboxy, carbalcoxy à radical alcoxy en $C_1$ à $C_4$ et alcanoyle en $C_1$ à $C_4$, ou un sel d'un azole à deux ou trois atomes d'azote, et comme agent antagoniste au moins une tétrahydro-1,3-oxazine suivant la revendication 1.

5. Composition herbicide suivant la revendication 4, caractérisé en ce qu'elle contient comme agent herbicide le 2-chloro-2',6'-diméthyl-N-(pyrazole-1-yl-méthyl)-acétanilide et comme agent antagoniste la N-dichloracétyl-4,4-diméthyl-tétrahydro-1,3-oxazine.

6. Composition herbicide suivant la revendication 4, caractérisée en ce qu'elle contient comme agent herbicide le 2-chloro-2',6'-diméthyl-N-(pyrazole-1-yl-méthyl)-acétanilide et comme agent antagoniste la N-dichloracétyl-4,4,6-triméthyl-tétrahydro-1,3-oxazine.

7. Composition herbicide suivant la revendication 4, caractérisée en ce que les proportions pondérales de l'acétanilide et de la tétrahydro-1,3-oxazine, appliqués ensemble ou séparément, sont dans un rapport compris entre 1:2 et 1:0,05.

8. Procédé de lutte sélective contre les plantes indésirables, caractérisé en ce que l'on applique avant, pendant ou après l'ensemencement des plantes cultivées ou avant ou après leur émergence, simultanément ou séparément dans un ordre quelconque, des acétanilides substitués de la formule générale I suivant la revendication 1 et des tétrahydro-1,3-oxazines de la formule II suivant la revendication 1.

9. Procédé de lutte sélective contre les plantes indésirables à l'aide d'acétanilides substituées de la formule I de la revendication 4, caractérisé en ce que les semences des plantes de culture sont traitées avec une ou plusieurs tétrahydro-1,3-oxazines de la formule II de la revendication 1.

10. Procédé suivant l'une des revendications 8 et 9, caractérisé en ce que la plante de culture est le maïs ou une céréale.

**Revendication AT**

1. Composition herbicide, caractérisée en ce qu'elle contient comme agent herbicide actif au moins un acétanilide substitué de la formule générale

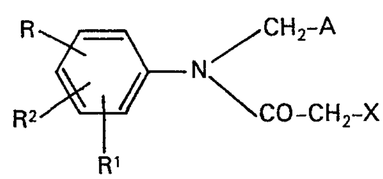

I

dans laquelle
R désigne un atome d'hydrogène ou un radical alcoyle ou alcoxy à chaîne droite ou ramifiée en $C_1$ à $C_5$;
$R^1$ désigne un atome d'hydrogène ou d'halogène ou un radical alcoyle ou alcoxy à chaîne droite ou ramifiée en $C_1$ à $C_5$;
$R^2$ désigne un atome d'hydrogène ou d'halogène ou un radical alcoyle ou alcoxy à chaîne droite ou ramifiée en $C_1$ à $C_5$ et forme avec R une chaîne alcoylène comprenant jusqu'à 6 atomes de carbone, éventuellement substituée par des radicaux alcoyle à chaîne droite ou ramifiée en $C_1$ à $C_4$, à connexion en position ortho;

X représente un atome de chlore ou de brome et A représente un groupe azole lié par un atome d'azote du noyau et qui peut porter un ou plusieurs substituants choisis parmi les atomes d'halogène et les groupes phényle, alcoyle, alcoxy, alcoyl-thio et perfluoralcoyle comportant jusqu'à 4 atomes de carbone, cyano, carboxy, carbalcoxy à radical alcoxy en $C_1$ à $C_4$ et alcanoyle en $C_1$ à $C_4$, ou un sel d'un azole à deux ou trois atomes d'azote, et comme agent antagoniste au moins une tétrahydro-1,3-oxazine de la formule générale

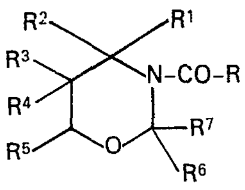

II

dans laquelle

R désigne un radical halo-alcoyle à chaîne droite ou ramifiée en $C_1$ à $C_3$;

$R^1$, $R^2$, $R^3$, $R^4$ et $R^5$, qui peuvent être identiques ou différents, désignent chacun un atome d'hydrogène ou un radical alcoyle à chaîne droite ou ramifiée en $C_1$ à $C_3$;

$R^6$ représente un atome d'hydrogène ou un radical alcoyle à chaîne droite ou ramifiée en $C_1$ à $C_8$;

$R^7$ représente un atome d'hydrogène, un radical alcoyle à chaîne droite ou ramifiée en $C_1$ à $C_8$, un radical alcoxy-alcoyle comprenant jusqu'à 6 atomes de carbone ou un radical dialcoxy-alcoyle comprenant jusqu'à 8 atomes de carbone,

$R^6$ et $R^7$ pouvant former ensemble une chaîne méthylène en $C_4$ ou $C_5$.

2. Composition herbicide suivant la revendication 1, caractérisée en ce qu'elle contient comme agent antagoniste la N-dichloracétyl-4,4-diméthyl-tétrahydro-1,3-oxazine.

3. Composition herbicide suivant la revendication 1, caractérisée en ce qu'elle contient comme agent antagoniste la N-dichloracétyl-4,4,6-triméthyl-tétrahydro-1,3-oxazine.

4. Composition herbicide suivant la revendication 1, caractérisée en ce qu'elle contient comme agent herbicide le 2-chloro-2',6'-diméthyl-N-(pyrazole-1-yl-méthyl)-acétanilide et comme agent antagoniste la N-dichloracétyl-4,4-diméthyl-tétrahydro-1,3-oxazine.

5. Composition herbicide suivant la revendication 1, caractérisée en ce qu'elle contient comme agent herbicide le 2-chloro-2',6'-diméthyl-N-(pyrazole-1-yl-méthyl)-acétanilide et comme agent antagoniste la N-dichloracétyl-4,4,6-triméthyl-tétrahydro-1,3-oxazine.

6. Composition herbicide suivant la revendication 1, caractérisée en ce que les proportions pondérales de l'acétanilide et de la tétrahydro-1,3-oxazine, appliqués ensemble ou séparément, sont dans un rapport compris entre 1:2 et 1:0,05.